# EUROPEAN PATENT APPLICATION

(11) **EP 4 831 294 A1**
(43) Date of publication of application: **28.10.2026**
(21) Application number: 24906886.7
(22) Date of filing: 11.10.2024
(51) Int. Cl.: C12P 7/625, C08L 101/16, C12N 15/09

(54) **METHOD FOR PRODUCING POLYHYDROXYALKANOIC ACID AGGREGATE**

(30) Priority: 18.12.2023 JP 2023212878
(71) Applicant: MITSUBISHI GAS CHEMICAL COMPANY, INC., Chiyoda-ku Tokyo 100-8324 (JP)
(72) Inventor: ITANI, Shunto, Tokyo 100-8324 (JP); YOSHIMURA, Takafumi, Niigata-shi, Niigata 950-3112 (JP); ABE , Hisaki, Niigata-shi, Niigata 950-3112 (JP)
(74) Representative: Müller-Boré & Partner Patentanwälte PartG mbB
(86) International application number: PCT/JP2024/036507
(87) International publication number: WO 2025/134498

(57) **Abstract**

Provided is a method for efficiently producing an aggregate of polyhydroxyalkanoic acid (PHA) with high purity from a culture solution containing microbial cells that intracellularly accumulated PHA. The aggregate of PHA is obtained by a very simple method of adding hydrogen peroxide and a surfactant to a culture solution containing PHA-accumulating microbial cells, followed by stirring under a predetermined condition.

## Description

### TECHNICAL FIELD

The present invention relates to a method of culturing a microorganism that intracellularly produces polyhydroxyalkanoic acid, and producing an aggregate of polyhydroxyalkanoic acid from the culture solution, and an aggregate obtained through the method.

### BACKGROUND ART

Polyhydroxyalkanoic acid (hereinafter, referred to as "PHA") is intracellularly produced and accumulated in many microbial species. PHA is biodegradable thermoplastic polyester and also has received attention as a plastic of nonpetroleum origin because of growing concern about environmental problems, and its practical use is expected. Representative examples of PHA include poly(3-hydroxybutyric acid) (PHB).

However, the development of an efficient production method at an industrial scale is necessary for the practical use. Since PHA produced by a microorganism usually forms granules which are intracellularly accumulated in the microorganism the step of separating and purifying PHA from microbial cells is necessary. That is, use as a plastic requires removing a component other than PHA as much as possible and enhancing the purity of PHA.

Various methods have been proposed as separation and purification methods for PHA. Examples thereof include a method based on the combination of disruption treatment and surfactant treatment of PHA-containing microbial cells (PTL 1), a method of performing heat treatment under an alkaline condition, followed by disruption treatment (PTL 2), and a method of separating PHA by solubilizing a biologically derived component other than PHA by treatment with sodium hypochlorite, an enzyme, or the like (PTL 3).

Examples of the approach of separating PHA from a suspension obtained by the treatment or the like include separation operations such as centrifugation and filtration, and drying operations such as spray drying. However, separating intracellularly produced PHA particles without aggregation results in an increased amount of fine powder, making handling of the product difficult.

For the handling problem, it is possible to aggregate PHA by the addition of a salt or the like. However, PHA with high purity is very difficult to aggregate and separate from an aqueous suspension rich in microorganism-derived components such as protein. Even use of, for example, aluminum sulfate, which is widely used in activated sludge treatment and the like cannot selectively aggregate PHA because almost all the components in the aqueous suspension are aggregated. Even if PHA can be selectively aggregated with a polymer flocculant or the like, such an additive is difficult to separate from PHA and decreases the quality of the aggregate as a polymer material.

Other examples of the method include a method of heating a PHA suspension (PTL 4), and a method of repeating heating and cooling (PTL 5), though a decrease in the molecular weight of PHA is problematic due to heating to around the melting point of PHA.

PHA with high purity is obtained through deposition by dissolving PHA in an organic solvent, followed by addition of a poor solvent or water. Lower-chain ketone (PTL 6), tetrahydrofuran (PTL 7), or the like can be used as an extraction solvent. However, the method based on dissolution and deposition disadvantageously requires a large amount of an organic solvent and decreases the molecular weight of PHA due to heating upon the dissolution of PHA.

A method for obtaining an aggregate of PHA has also been reported which involves adjusting the pH of an aqueous PHA suspension to an acidic range after a purification step of removing microbial cell-derived impurities from a microbial cell culture solution to a predetermined concentration or below (PTL 8). This method has the advantages that the amount of organic solvent used is reduced and that the method involves neither the addition of a salt, a polymer flocculant, or the like, nor high-temperature treatment; however, a purification step is required, and further improvement in efficiency as an industrial process is needed. Further improvement in handling is also considered necessary.

### CITATION LIST

### PATENT LITERATURE

PTL 1: Japanese Translation of PCT International Application Publication No. 1996-502415
PTL 2: International Publication No. WO 2004/065608
PTL 3: Japanese Patent Laid-Open No. 2005-348640
PTL 4: Japanese Translation of PCT International Application Publication No. 2000-502399
PTL 5: Japanese Translation of PCT International Application Publication No. 2002-517582
PTL 6: Japanese Translation of PCT International Application Publication No. 1998-504460
PTL 7: Japanese Patent Laid-Open No. 07-79788
PTL 8: International Publication No. WO 2010-067543

### SUMMARY OF INVENTION

### TECHNICAL PROBLEM

Although various methods have been reported as described above, no method for efficiently obtaining an aggregate of PHA particles with high purity has been known for an industrial process. This is presumably partly because parameters governing the aggregation of PHA have remained unknown.

An object of the present invention is to provide a method for efficiently producing an aggregate of PHA with high purity from a culture solution containing intracellularly accumulated PHA. Another object thereof is to provide an aggregate of PHA with high purity by such an efficient method.

### SOLUTION TO PROBLEM

The present inventors have conducted diligent studies to attain the objects and found a solution. Specifically, it has been found that an aggregate of PHA is obtained by a very simple method of adding hydrogen peroxide and a surfactant to a culture solution containing a microbial cell containing PHA, followed by stirring under a condition of pH controlled at 8 or less at a required stirring power, as calculated from the Kamei-Hiraoka equation, of 2 W/m³ or more.

The present invention more specifically provides the following.
(1) A method for producing an aggregate of polyhydroxyalkanoic acid, the method comprising:
   a culture step of culturing a microorganism that intracellularly accumulates polyhydroxyalkanoic acid; and
   a stirring step of adding hydrogen peroxide and a surfactant to the obtained culture solution,
   followed by stirring under a condition of pH controlled at 8 or less at a required stirring power, as calculated from the Kamei-Hiraoka equation, of 2 W/m³ or more,
   wherein the surfactant is added in an amount of twice or more by weight relative to the microbial cell weight, which is calculated by subtracting the dry weight of polyhydroxyalkanoic acid from the dry cell weight of the microorganism including polyhydroxyalkanoic acid.
(2) The method according to (1), further comprising a collection step of collecting the aggregate of polyhydroxyalkanoic acid after the stirring step.
(3) The method according to (1) or (2), further comprising a sterilization step of performing sterilization treatment of the microorganism by heat treatment after the culture step.
(4) The method according to any of (1) to (3), wherein the pH in the stirring step is controlled at from 4.0 to 7.0.
(5) The method according to any of (1) to (4), wherein the surfactant is sodium dodecyl sulfate.
(6) The method according to any of (1) to (5), wherein an amount of hydrogen peroxide added is 2 to 10% by weight.
(7) The method according to any of (1) to (6), wherein the required stirring power calculated from the Kamei-Hiraoka equation is 5 W/m³ or more for 5 hours or longer after addition of hydrogen peroxide and the surfactant.
(8) The method according to any of (1) to (6), wherein the required stirring power calculated from the Kamei-Hiraoka equation is 10 W/m³ or more for 5 hours or longer after addition of hydrogen peroxide and the surfactant.
(9) The method according to any of (1) to (8), wherein a liquid temperature in the stirring step is 40°C to 100°C.
(10) The method according to any of (1) to (8), wherein a liquid temperature in the stirring step is 50°C to 80°C.
(11) The method according to any of (1) to (10), wherein a median size of the obtained aggregate of polyhydroxyalkanoic acid is 1000 µm or more.
(12) The method according to any of (1) to (11), wherein a total nitrogen concentration of the obtained aggregate of polyhydroxyalkanoic acid is 500 ppm or lower.
(13) The method according to any of (1) to (12), wherein the polyhydroxyalkanoic acid contains 3-hydroxybutyric acid as a monomer unit.
(14) The method according to any of (1) to (13), wherein the polyhydroxyalkanoic acid is a copolymer containing 3-hydroxybutyric acid and 4-hydroxybutyric acid as monomer units.
(15) The method according to any of (1) to (14), wherein the microorganism is a microorganism of a genus selected from the group consisting of the genus *Cupriavidus,* the genus *Alcaligenes,* the genus *Ralstonia,* the genus *Delftia,* the genus *Comamonas,* the genus *Hydrogenophaga,* the genus *Burkholderia,* the genus *Escherichia,* the genus *Azotobacter,* the genus *Methylobacterium,* the genus *Paracoccus,* the genus *Pseudomonas,* the genus *Acinetobacter,* the genus *Aeromonas,* the genus *Allochromatium,* the genus *Azorhizobium,* the genus *Bacillus,* the genus *Caulobacter,* the genus *Chromobacterium,* the genus *Ectothiorhodospira,* the genus *Klebsiella,* the genus *Nocardia,* the genus *Rhodobacter,* the genus *Rhodococcus,* the genus *Rhodospirillum,* the genus *Rickettsia,* the genus *Sinorhizobium,* the genus *Sphingomonas,* the genus *Synechocystis,* the genus *Thiococcus,* the genus *Thiocystis,* the genus *Vibrio,* and the genus *Wautersia.*
(16) The method according to (15), wherein the microorganism is *Cupriavidus necator.*
(17) A polyhydroxyalkanoic acid aggregate having a median size of 1000 µm or more and a bulk density of 0.06 g/mL to 0.20 g/mL in a dry state.
(18) The polyhydroxyalkanoic acid aggregate according to (17), wherein the bulk density in a dry state is 0.08 g/mL to 0.10 g/mL.
(19) The polyhydroxyalkanoic acid aggregate according to (17) or (18), wherein a total nitrogen concentration is 500 ppm or lower.

### ADVANTAGEOUS EFFECTS OF INVENTION

According to the present invention, a PHA aggregate can be produced by a simple method from a microbial cell culture solution containing PHA. The method of the present invention is particularly beneficial for an industrial process by drastically reducing the complexity of the process. Aggregates with excellent handleability and large particle sizes are obtained, and this is also highly advantageous for subsequent processing steps. Furthermore, the amount of impurities in the PHA aggregate of the present invention is small and the PHA aggregate can be used for various purposes, including medical applications.

### BRIEF DESCRIPTION OF DRAWINGS

Fig. 1 shows a scanning electron microscope photograph of a PHA aggregate obtained by the method of the present invention;

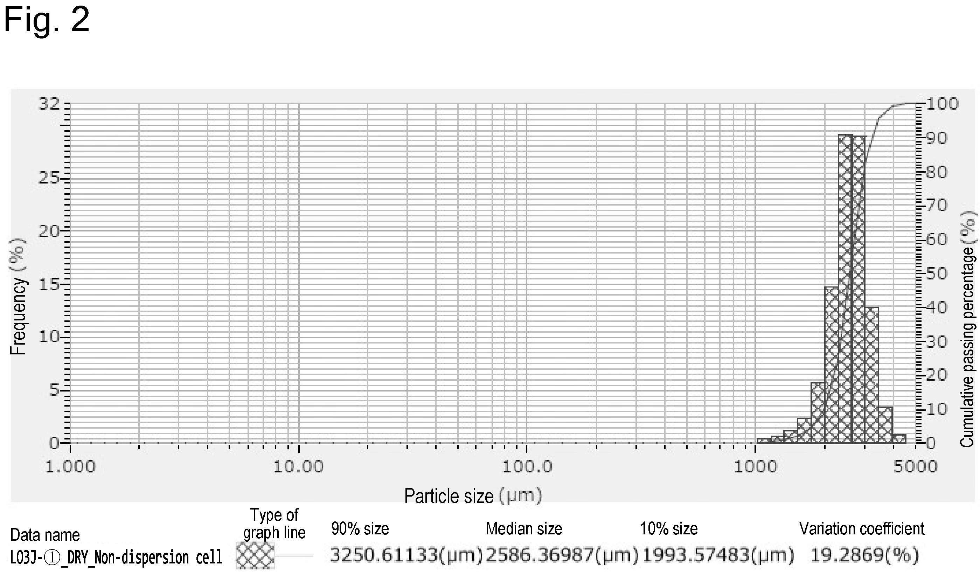

Fig. 2 shows a chart showing the particle size and the coefficient of variation of a PHA aggregate obtained through a single stirring step in experiment 1, as measured in a dry system using a particle size analyzer (manufactured by HORIBA, Ltd., LA-960N2);

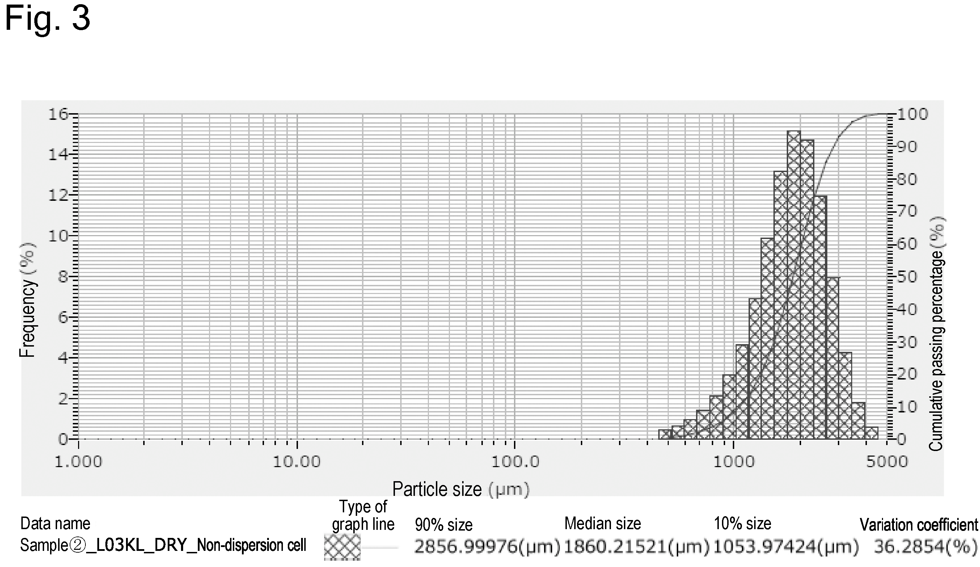

Fig. 3 shows a chart showing the particle size and the coefficient of variation of a PHA aggregate obtained in experiment 2, as measured in a dry system using a particle size analyzer (manufactured by HORIBA, Ltd., LA-960N2);

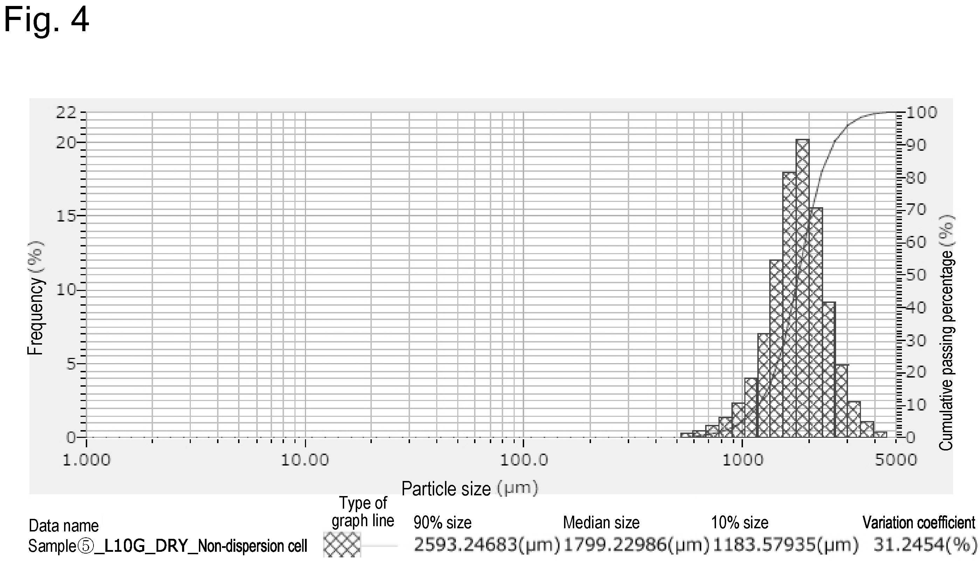

Fig. 4 shows a chart showing the particle size and the coefficient of variation of a PHA aggregate obtained in experiment 3, as measured in a dry system using a particle size analyzer (manufactured by HORIBA, Ltd., LA-960N2);

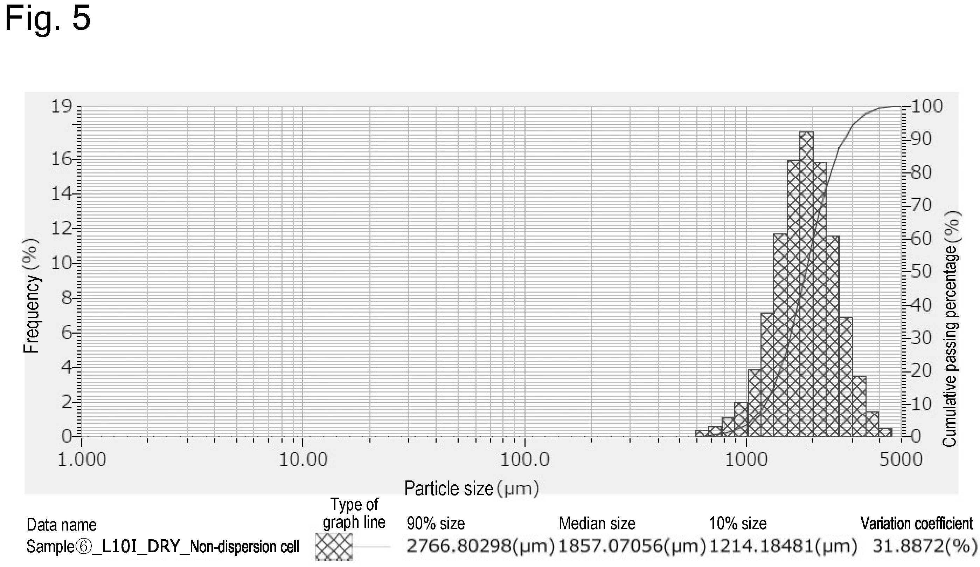

Fig. 5 shows a chart showing the particle size and the coefficient of variation of a PHA aggregate obtained in experiment 4, as measured in a dry system using a particle size analyzer (manufactured by HORIBA, Ltd., LA-960N2);

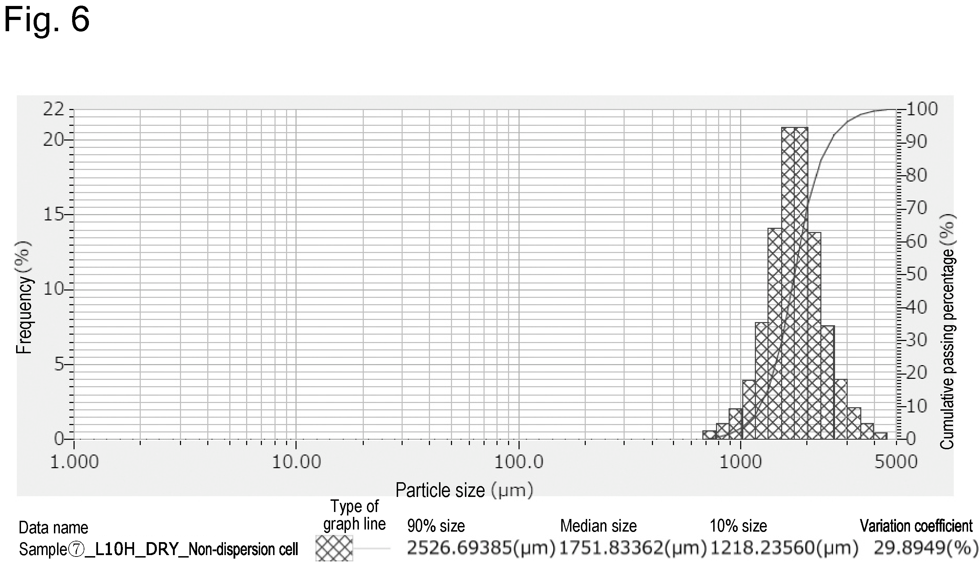

Fig. 6 shows a chart showing the particle size and the coefficient of variation of a PHA aggregate obtained in experiment 5, as measured in a dry system using a particle size analyzer (manufactured by HORIBA, Ltd., LA-960N2);

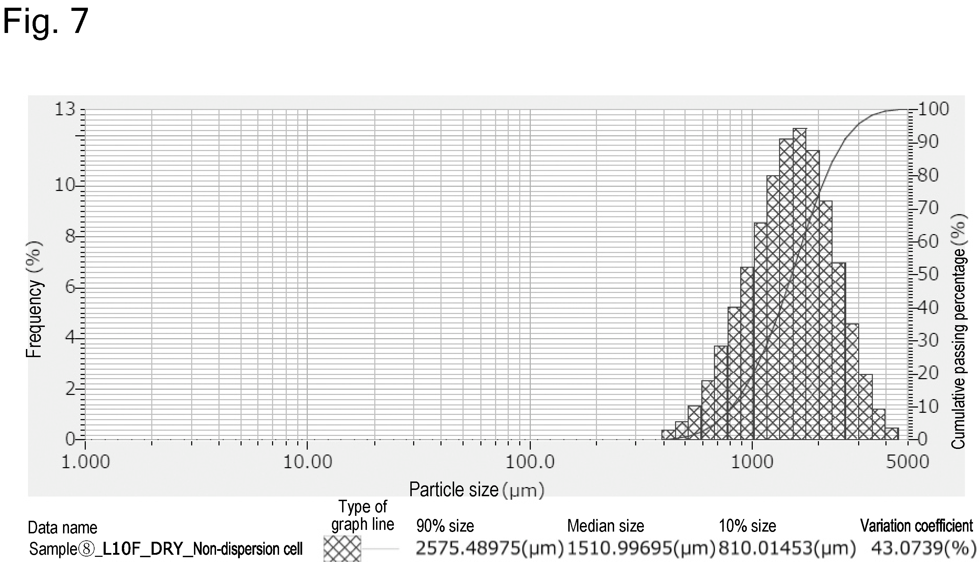

Fig. 7 shows a chart showing the particle size and the coefficient of variation of a PHA aggregate obtained in experiment 6, as measured in a dry system using a particle size analyzer (manufactured by HORIBA, Ltd., LA-960N2);

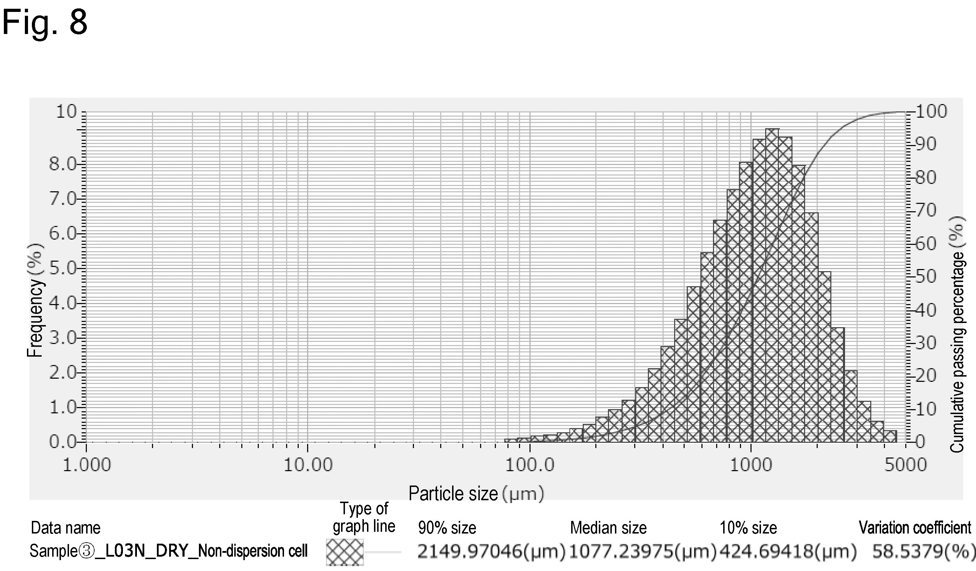

Fig. 8 shows a chart showing the particle size and the coefficient of variation of a PHA aggregate obtained in experiment 7, as measured in a dry system using a particle size analyzer (manufactured by HORIBA, Ltd., LA-960N2);

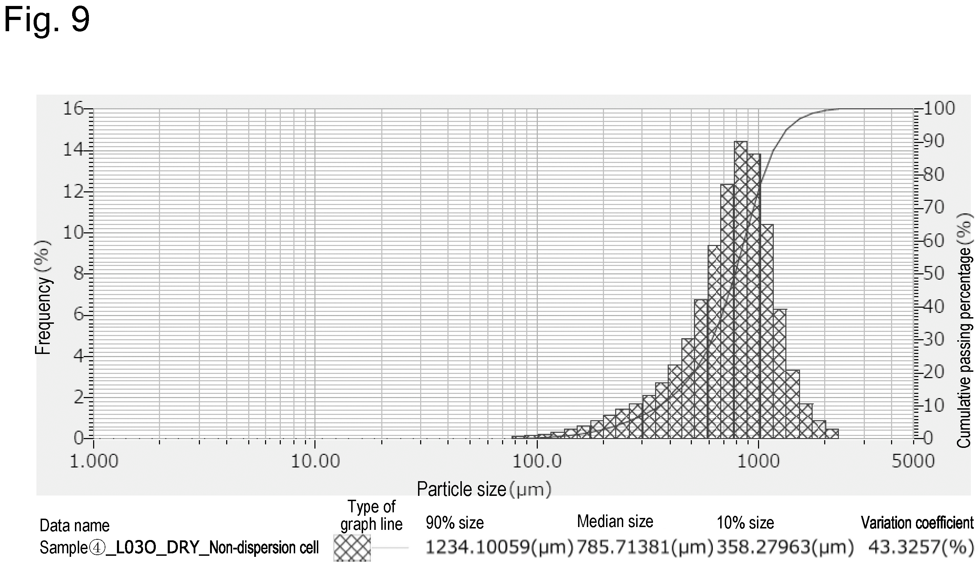

Fig. 9 shows a chart showing the particle size and the coefficient of variation of a PHA aggregate obtained in experiment 8, as measured in a dry system using a particle size analyzer (manufactured by HORIBA, Ltd., LA-960N2);

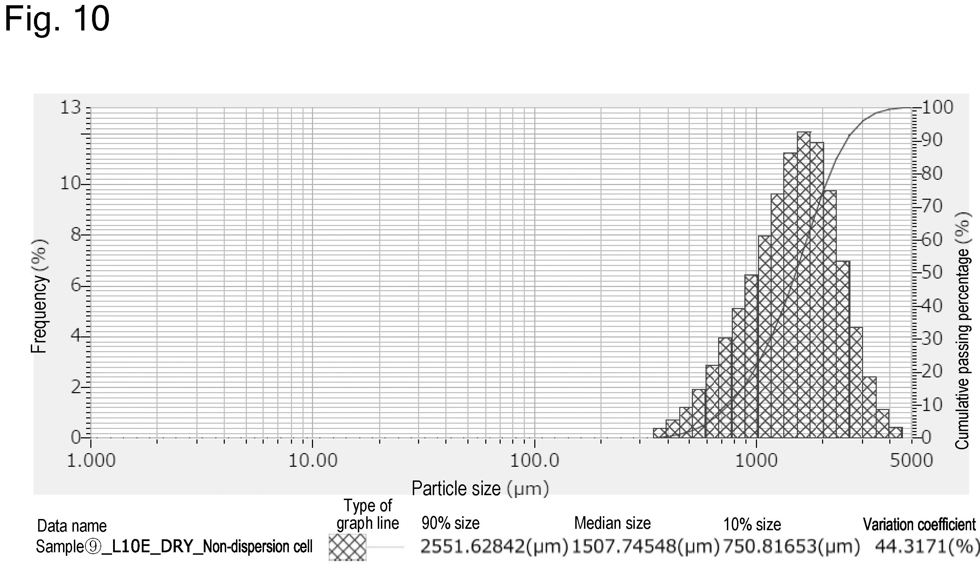

Fig. 10 shows a chart showing the particle size and the coefficient of variation of a PHA aggregate obtained in experiment 10, as measured in a dry system using a particle size analyzer (manufactured by HORIBA, Ltd., LA-960N2); and

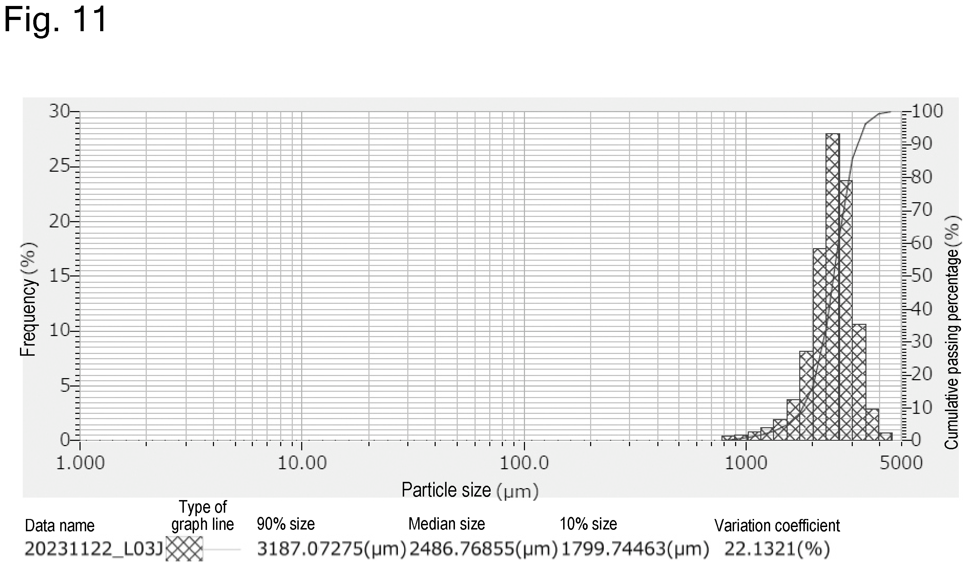

Fig. 11 shows a chart showing the particle size and the coefficient of variation of a PHA aggregate obtained through two stirring steps in experiment 1, as measured in a dry system using a particle size analyzer (manufactured by HORIBA, Ltd., LA-960N2).

### DESCRIPTION OF EMBODIMENTS

Hereinafter, the method and the like according to the present invention will be specifically described.

The method of the present invention includes the steps of: culturing a microorganism that intracellularly accumulates polyhydroxyalkanoic acid; and adding hydrogen peroxide and a surfactant to the obtained culture solution, followed by stirring under a condition of pH controlled at 8 or less at a required stirring power, as calculated from the Kamei-Hiraoka equation, of 2 W/m³ or more.

### <PHA>

PHA according to the present invention is a generic name for polymers having 3-hydroxyalkanoic acid or 4-hydroxyalkanoic acid as a monomer unit. Examples of the constituent hydroxyalkanoic acid include, but are not particularly limited to, 3-hydroxypropionate, 3-hydroxybutyrate, 3-hydroxyvalerate, 3-hydroxyhexanoate, 3-hydroxyheptanoate, 3-hydroxyoctanoate, and 4-hydroxybutyrate. PHA includes not only a homopolymer but a copolymer constituted by two or more hydroxyalkanoic acids selected from the group consisting of 3-hydroxypropionate, 3-hydroxybutyrate, 3-hydroxyvalerate, 3-hydroxyhexanoate, 3-hydroxyheptanoate, 3-hydroxyoctanoate, and 4-hydroxybutyrate.

The copolymer is preferably a two-component copolymer of 3-hydroxybutyrate (3HB) and 4-hydroxybutyrate (4HB) in terms of physical properties. The composition ratio of each monomer unit constituting the two-component copolymer of 3HB and 4HB is not particularly limited; however, the 4HB unit is 1 to 99 mol%, preferably 1 to 50 mol%, more preferably 5 to 25 mol%, when the total of all the monomer units is defined as 100 mol%.

### <Culture step>

In the method of the present invention, a microorganism that accumulates PHA is used. The microorganism to be used is not particularly limited as long as it accumulates PHA. For example, a microorganism isolated from nature, a microorganism deposited in a microbial or bacterial strain depository (e.g., IFO or ATCC), or a mutant or transformant that may be prepared therefrom can be used. Examples of such microorganisms include those genus *Cupriavidus,* the genus *Alcaligenes,* the genus *Ralstonia,* the genus *Pseudomonas,* the genus *Bacillus,* the genus *Azotobacter,* the genus *Nocardia,* and the genus *Aeromonas.* Among them, a microorganism belonging to the genus *Aeromonas,* the genus *Alcaligenes,* the genus *Ralstonia,* or the genus *Cupriavidus* is preferred. Particularly, a microbial strain such as *Alcaligenes lipolytica, Alcaligenes latus, Aeromonas caviae, Aeromonas hydrophila,* or *Cupriavidus necator* is more preferred, and *Cupriavidus necator* is most preferred.

When the microorganism intrinsically has no ability to produce PHA or produces PHA in a small amount, a transformant obtained by introducing a synthase gene for the PHA of interest and/or mutants thereof to the microorganism may be used.

In the culture step, a PHA-accumulating microorganism is cultured in a medium containing nutrients. The medium for use in culture can be used without limitations as long as the medium enables the microorganism to proliferate. For example, a medium containing a carbon source such as glucose, fructose, methanol, acetic acid, or butyric acid, a nitrogen source such as ammonium sulfate, ammonium nitrate, or peptone, a phosphate source such as potassium phosphate or sodium phosphate, various minerals, and a micronutrient can be used.

An antifoaming agent may be added to suppress foaming.

Culture conditions under which the microorganism used is capable of proliferating and capable of accumulating PHA can be employed. From this viewpoint, a culture time, an aeration condition, and the like can be appropriately set. The PHA content of the microbial cell of the microorganism after the completion of culture is not particularly limited and is preferably 20% or more, more preferably 30% or more, further preferably 40% or more, still further preferably 50% or more.

After the completion of culture, heat sterilization may be performed for several hours at a temperature that kills the microorganism.

### <Stirring step>

The stirring step is performed by adding hydrogen peroxide and a surfactant.

The surfactant used is not particularly limited and is preferably an anionic surfactant, particularly preferably sodium dodecyl sulfate. The concentration of the surfactant used is preferably at least twice, more preferably at least three times, particularly preferably at least four times the net dry cell weight (the cell weight calculated by subtracting the dry weight of PHA from the dry cell weight of the microorganism including PHA) by weight. The upper limit is not particularly limited and is preferably a weight of 10 times or less in terms of cost. Thus, the concentration of the surfactant added is determined by measuring the dry microbial cell weight including PHA and the dry weight of PHA; alternatively, the concentration of the surfactant added may be simply determined from an estimated value of the net dry microbial cell weight. For example, when the net dry microbial cell weight is estimated as 10 g/L, the amount of the surfactant added can be in the range of 2% by weight to 10% by weight.

The amount of hydrogen peroxide added is 2 to 10% by weight based on the amount of the solution, and is preferably 4 to 8% by weight. The addition may be performed at once or in multiple divided portions.

The pH of the solution in the stirring step is preferably controlled at 8 or less. More preferably, the pH is controlled at from 4.0 to 7.0. A liquid temperature in the stirring step is preferably 40 to 100°C, more preferably 50 to 80°C.

Examples of the type of a stirring blade include, but are not limited to, flat paddle-type blades, inclined paddle blades, Rushton turbine (disc turbine) blades, propeller blades, 3-blade retreat (Pfaudler) impellers, anchor blades, helical ribbon blades, and screw blades. The required stirring power calculated from the Kamei-Hiraoka equation is preferably 2 W/m³ or more, more preferably 5 W/m³ or more, further preferably 10 W/m³ or more, particularly preferably 20 W/m³ or more.

An antifoaming agent may be added to suppress foaming.

The stirring step may be carried out twice or more, for example, to further remove microbial cell-derived impurities. The stirring step in the second and subsequent runs can be performed under the same conditions as those of the first run, or may be performed under different conditions. For example, in the stirring step in the second and subsequent runs, the surfactant may not be used, and the stirring time may be shorter than that of the first run. By carrying out the stirring step twice or more, a PHA aggregate with higher purity can be obtained, and the size (median particle size) and shape of the aggregate are maintained.

### <PHA aggregate collection step>

The PHA aggregate can be appropriately collected from the solution after the stirring step by filtration or the like. The resulting aggregate has a large particle size and therefore has excellent handleability and is easy to collect. If necessary, the collected PHA may be washed with water or the like and then dried to obtain a dry aggregate.

### <PHA aggregate>

In the method of the present invention, a high-purity the PHA aggregate can be produced by a simple method from a microbial cell culture solution containing PHA. The amount of organic nitrogen contained in the PHA aggregate can be measured using, for example, a trace total nitrogen analyzer (manufactured by Nittoseiko Analytech Co., Ltd., TN-2100H). The-organic nitrogen content of-the PHA aggregate is 500 ppm or lower, preferably 400 ppm or lower, more preferably 300 ppm or lower, further preferably 200 ppm or lower, particularly preferably 100 ppm or lower.

In the present invention, the PHA aggregate can be prepared by a simple method, and a decrease in molecular weight of PHA is also suppressed during the-separation and purification process. The molecular weight of the PHA aggregate is preferably 500000 or greater, more preferably 600000 or greater, further preferably 700000 or greater.

The particle size of the PHA aggregate can be measured in a dry system using, for example, a particle size analyzer (manufactured by HORIBA, Ltd., LA-960N2). The median size of the PHA aggregate is preferably 1000 µm or more, and the 10% (D10) particle size is preferably 500 µm or more. The upper limit is not particularly limited, and a PHA aggregate having a median size of 10 mm or less can be obtained according to the present invention.

An aggregate having a large particle size is obtained, which is therefore highly advantageous in terms of handleability in a subsequent processing steps.

Furthermore, the PHA aggregate of the present invention has a small amount of impurities and can be used for various applications, including medical applications.

### EXAMPLES

Hereinafter, the present invention will be described in more detail with reference to Examples. However, the present invention is not limited to these Examples.

### (Method for measuring viscosity)

Viscosity measurement was performed by the following method.

A liquid sample was collected into a 1 L container. A rotor (No. 19L/ADP) was attached to a viscometer (TVB-10M manufactured by Toki Sangyo Co., Ltd.), and the rotor was immersed in the-sample liquid, and rotated at 100 rpm at a liquid temperature of 65°C to measure a viscosity.

### (Method for calculating required stirring power)

The required stirring power (P) is calculated according to the following equation.$P \left(kW\right) = {N}_{p} {ρn}^{3} {d}^{5}$ (Nₚ : Power number, ρ: Liquid density, n: Rotational speed (rpm/60), d: Blade diameter) ${N}_{p} = A / Re + B \left(Re:Reynolds number\right)$ Re = nd² ρ/µ (µ:Liquid viscosity, ρ: Liquid density, n: Rotational speed (rpm/60), d: Blade diameter)

The power number (Np) was calculated from the following Kamei-Hiraoka equation.${N}_{po} = \left\{\left[1.2{π}^{4}{β}^{2}\right]/\left[8{d}^{3}/\left({D}^{2}H\right)\right]\right\} f$ $f = {C}_{L} / {Re}_{G} + {C}_{t} {\left\{{\left[\left({C}_{tr}/{Re}_{G}\right)+{Re}_{G}\right]}^{- 1}+{\left({f}_{∼}/{C}_{t}\right)}^{1 / m}\right\}}^{m}$ ${Re}_{d} = {nd}^{2} ρ / μ$ ${Re}_{G} = \left\{\left[πηln\left(D/d\right)\right]/\left(4d/βD\right)\right\} {Re}_{d}$ ${C}_{L} = 0.215 {ηn}_{p} \left(d/H\right) \left[1-{\left(d/D\right)}^{2}\right] + 1.83 \left(bsinθ/H\right) {\left({n}_{p}/2sinθ\right)}^{1 / 3}$ ${C}_{t} = {\left[{\left(1.96{X}^{1.19}\right)}^{- 7.8}+{\left(0.25\right)}^{- 7.8}\right]}^{- 1 / 7.8}$ $m = {\left[{\left(0.71{X}^{0.373}\right)}^{- 7.8}+{\left(0.333\right)}^{- 7.8}\right]}^{- 1 / 7.8}$ ${C}_{tr} = 23.8 {\left(d/D\right)}^{- 3.24} {\left(bsinθ/D\right)}^{- 1.18} {X}^{- 0.74}$ ${f}_{∼} = 0.0151 \left(d/D\right) {{C}_{t}}^{0.308}$ $X = {{γn}_{p}}^{0.7} b {\left(sin\frac{θ}{H}\right)}^{1.6}$ $β = 2 ln \left(D/d\right) / \left[\left(D/d\right)-\left(d/D\right)\right]$ $γ = {\left[ηln\left(D/d\right)/\left(βD/{d}^{5}\right)\right]}^{1 / 3}$ $η = 0.711 \left\{0.157+{n}_{p} ln{\left(D/d\right)}^{0.611}\right\} / \left\{{{n}_{p}}^{0.52}\left[1-{\left(d/D\right)}^{2}\right]\right\}$

Apparatus conditions are as follows.
d: blade diameter = 0.168 m
b: blade width = 0.025 m
D: tank diameter = 0.33 m
H: liquid depth = 0.093 m
nₚ: the number of blades = 2
θ: angle of blade attachment = 90 deg

### (Method for measuring bulk density)

Bulk density measurement was performed by the following method.

The dried PHA aggregate was sifted through a sieve having an opening of 4 mm to remove aggregates of 4 mm or more in size. Then, 100 mL of the PHA aggregate was placed into a 100 mL graduated cylinder, and the weight of the PHA aggregate in the cylinder was measured using a precision balance. The weight (g) was divided by 100 (mL) to calculate the bulk density (g/mL).

### <Experiment 1>

### [Preparation of culture seed]

Preparation of culture solution containing PHA consisting of 3-hydroxybutyric acid and 4-hydroxybutyric acid (P(3HB-co-4HB)) using *Cupriavidus necator* H16 strain (ATCC17696)

Fructose and ε-caprolactone were added at 7.25 g/L and 5 g/L, respectively, to six 1 L Erlenmeyer flasks each containing 420 mL of a sterilized medium composed of 2.72 g/L KH₂PO₄, 4.26 g/L Na₂HPO₄, 0.3 g/L NaHCO₃, 2 g/L (NH₄)₂SO₄, 0.2 g/L MgSO₄·7H₂O, 0.2 g/L yeast extract, and 3.5 mL/L mineral solution. Then, 2 mL of a frozen microbial cell suspension was inoculated thereto, and the mixture was cultured at 252 rpm at 30°C for 96 hours to prepare a seed culture (preculture solution).

The mineral solution described above is as follows:
6 g/L FeC₆H₅O₇·xH₂O, 2 g/L ZnSO₄·7H₂O, 0.1 g/L CuSO₄·5H₂O, 1 g/L MnCl₂·4H₂O, 0.1 g/L KI, 0.1 g/L (NH₄)₆Mo₇O₂₄·4H₂O, 0.1 g/L CoCl₂·6H₂O, 0.2 g/L H₃BO₃, 5 g/L NaCl, and 4 g/L CaCl₂·2H₂O were dissolved in water for preparation.

### [Main culture]

250 L of a medium was prepared in a 500 L jar fermenter by changing the amount of (NH₄)₂SO₄ in medium 1 described above to 7.3 g/L and adding Pluronic L101 (antifoaming agent: manufactured by ADEKA corp.) at 0.08 wt%, and sterilized, and 2.52 L of the seed culture was inoculated thereto. The feeding of a 42% by mass of fructose solution and ε-caprolactone was aseptically started via a sterilization filter (PTFE, 0.2 µm pore). Although the feeding rates and the feeding ratios of the carbon sources can be arbitrarily set, the culture was started at low flow rates at a fructose solution feeding rate of 0.2 to 2.0 g/(h·L) and a ε-caprolactone feeding rate of 0.03 to 0.4 g/h·L, and the feeding rates were increased in stages or continuously depending on microbial cell proliferation, in order to prevent the carbon sources from remaining in excess in the culture vessel without being completely consumed by the microorganism, which would lead to arrest of microbial cell proliferation. The aeration rate was 50 to 100 L/min, the stirring speed was 130 to 153 rpm, the culture temperature was 36°C, and the culture pH was controlled at a lower limit of 6.0. A 4 M aqueous sodium hydroxide solution was used as an alkali for pH adjustment. The culture was terminated approximately 170 hours after the start of culture.

The obtained culture solution was heated-sterilized with stirring at 60°C for 80 minutes.

During or after culture, microbial cells and the culture supernatant were separated by centrifugation, and the microbial cells were frozen at -20°C and then freeze-dried.

The freeze-dried microbial cells were used for PHA composition analysis and PHA molecular weight analysis.

The composition analysis of PHA and the PHA content in the microbial cells were determined by methylesterification followed by gas chromatography analysis of methyl ester derivatives derived from the monomer units constituting PHA.

A portion of the microbial cell culture solution subjected to the sterilization treatment described above was sampled, and the supernatant was removed by centrifugation to collect microbial cells and PHA. These were frozen at -20°C and then subjected to freeze-dried, and the total nitrogen concentration was measured using an organic element analyzer (manufactured by Thermo fisher Scientific Inc., FlashSmart). As a result, the organic nitrogen concentration in the aqueous PHA suspension was 63,500 ppm based on the polymer weight.

### [Production of PHA aggregate]

The microbial cell culture solution subjected to the sterilization treatment described above was transferred to a glass-lined 30 L reaction vesse, and the reaction vessel was immersed in a hot water bath set to 75.0°C to start temperature elevation of the culture solution. At the point in time when the internal temperature reached 70°C, sodium dodecyl sulfate (hereinafter, referred to as "SDS") was added in an amount of four times by weight relative to the net dry cell weight (the cell weight calculated by subtracting the weight of polyhydroxyalkanoic acid from the cell weight including polyhydroxyalkanoic acid). Subsequently, 35% hydrogen peroxide solution was added thereto at 6% by weight. The time at which the addition of hydrogen peroxide solution was completed was defined as the start of the reaction, and the reaction was performed for 21 hours. The temperature was maintained at approximately 70°C from the start of heating to the completion of the reaction. Stirring was performed at a required stirring power of 120 W/m³ calculated from the Kamei-Hiraoka equation, and pH was controlled at 6.0 by adding a 15% by weight aqueous sodium hydroxide solution.

Five hours after the start of the reaction, the reaction liquid was sampled, and sulfuric acid (1:4 v/v, acid:water) was added thereto, followed by determination of hydrogen peroxide concentration by oxidation-reduction titration using a 0.02 mol/L potassium permanganate solution. Hydrogen peroxide was added to the reaction liquid to compensate for the consumed amount such that the hydrogen peroxide concentration was 6% by weight.

Twenty-one hours after the start of the reaction, the reaction liquid was subjected to solid-liquid separation using a filter cloth having an air permeability of 15 cc/cm²/sec to collect a PHA aggregate. The collected PHA was washed with pure water and dried, and the recovery rate was calculated. $Collection rate = \frac{Amount of PHA collected \left(g\right)}{Amount of culture liquid \left(L\right) \times Microbial cell concentration \left(g/L\right) \times PHA content \left(%\right)} \times 100$

In Experiment 1, a second stirring step was performed.

The collected PHA and 5 L of pure water were transferred to a 10 L medium bottle, and the medium bottle was immersed in a hot water bath set to 75.0°C to start temperature elevation of the aqueous PHA suspension. At the point in time when the internal temperature reached 70°C, a 35% hydrogen peroxide solution was added thereto a final concentration of 6% by weight. The time at which the addition of the hydrogen peroxide solution was completed was defined as the start of reaction, and the reaction was performed for 7 hours. The temperature was maintained at approximately 70°C from the start of heating to just before the completion of the reaction. pH was controlled at 7.5 by adding a 5% by weight aqueous sodium hydroxide solution.

Seven hours after the start of reaction, solid-liquid separation of the reaction liquid was performed using a filter cloth having an air permeability of 15 cc/cm²/sec to collect a PHA aggregate. The collected PHA was washed with pure water until the hydrogen peroxide concentration in the filtrate was reduced to 2% or less, then washed with ethanol, and subsequently dried, and a recovery rate was calculated.

The dried PHA aggregate-was sifted through a sieve having an opening of 4 mm to remove aggregates of 4 mm or more in size. Subsequently, the particle size and the coefficient of variation of the PHA aggregate were measured in a dry system using a particle size analyzer-(manufactured by HORIBA, Ltd., LA-960N2).

The total nitrogen concentration of the PHA aggregate was measured using a trace total nitrogen analyzer (manufactured by Nittoseiko Analytech Co., Ltd., TN-2100H).

### <Experiment 2>

Experiment 2 was conducted in the same manner as Experiment 1, except that the required stirring power in the stirring step, as calculated from the Kamei-Hiraoka equation, was set to 18 W/m³, and a single stirring step was performed.

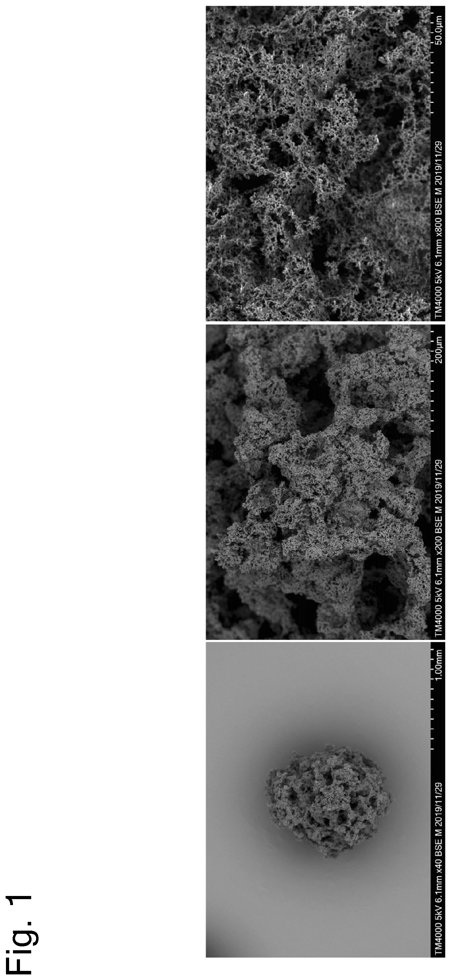

Fig. 1 shows a scanning electron microscopy (SEM) image of the obtained PHA aggregate.

### <Experiment 3>

Experiment 3 was conducted in the same manner as Experiment 1, except that the amount of SDS added in the stirring step was set to three times by weight relative to the net dry cell weight, the required stirring power in the stirring step, as calculated from the Kamei-Hiraoka equation, was set to 831 W/m³; and the single stirring step was performed.

### <Experiment 4>

Experiment 4 was conducted in the same manner as Experiment 1, except that the pH in the stirring step was controlled at 4.0, the required stirring power in the stirring step, as calculated from the Kamei-Hiraoka equation, was set to 831 W/m³, and a single stirring step was performed.

### <Experiment 5>

Experiment 5 was conducted in the same manner as Experiment 1, except that the pH in the stirring step was controlled at 5.0, the required stirring power in the stirring step, as calculated from the Kamei-Hiraoka equation, was set to 831 W/m³, and a single stirring step was performed.

### <Experiment 6>

Experiment 6 was conducted in the same manner as Experiment 1, except that the pH in the stirring step was controlled at 7.0, and a single stirring step was performed.

### <Experiment 7>

Experiment 7 was conducted in the same manner as Experiment 1, except that the required stirring power in the stirring step, as calculated from the Kamei-Hiraoka equation, was set to 2.7 W/m³, and a single stirring step was performed.

### <Experiment 8>

Experiment 8 was conducted in the same manner as Experiment 1, except that the required stirring power in the stirring step, as calculated from the Kamei-Hiraoka equation, was set to 0.7 W/m³, and a single stirring step was performed.

### <Experiment 9>

Experiment 9 was conducted in the same manner as Experiment 1, except that the amount of SDS added in the stirring step was set to be equal in weight to the net dry cell weight, and a single stirring step was performed.

### <Experiment 10>

Experiment 10 was conducted in the same manner as Experiment 1, except that the pH in the stirring step was controlled at 7.5, and a single stirring step was performed.

Tables 1 and 2 show detailed conditions and measurement results of each experimental system.

In the experiments except for Experiment 9, a PHA aggregate with high purity was obtained. Particularly, in Experiments 1 to 3, 5, and 6, a PHA aggregate with high purity having a median size of 1500 µm or more and a total nitrogen concentration of 100 ppm was obtained. It was therefore confirmed that a PHA aggregate with high purity was obtained by a very simple method.

In Experiment 1, the stirring step was performed twice. Table 3 shows the median size, the total nitrogen concentration, and other properties of the obtained PHA aggregate. It was confirmed that the stirring step twice drastically decreased the total nitrogen concentration, while the median size of the PHA aggregate remained largely unchanged.

**[Table 1]**

| | Type of polymer | Dry microbial cell concentration (g/L) | PHA content(%) | Net microbial cell concentration (g/L) | Reaction temperature (°C) | pH | Surfactant | Amount of surfactant | Hydrogen peroxide concentration (%) | Stirring speed (rpm) | Reaction solution density (g/mL) | Initial viscosity (cP) |
|---|---|---|---|---|---|---|---|---|---|---|---|---|
| Experiment 1 | P(3HB-co-4HB) | 24.7 | 63.0 | 9.13 | 70 | 6.0 | SDS | 4 times | 6.0 | 200 | 1.01 | 1.68 |
| Experiment 2 | P(3HB-co-4HB) | 24.7 | 63.0 | 9.13 | 70 | 6.0 | SDS | 4 times | 6.0 | 100 | 1.01 | 1.68 |
| Experiment 3 | P(3HB-co-4HB) | 25.1 | 56.3 | 10.95 | 70 | 6.0 | SDS | 3 times | 6.0 | 400 | 1.02 | 1.72 |
| Experiment 4 | P(3HB-co-4HB) | 25.1 | 56.3 | 10.95 | 70 | 4.0 | SDS | 4 times | 6.0 | 400 | 1.02 | 1.72 |
| Experiment 5 | P(3HB-co-4HB) | 25.1 | 56.3 | 10.95 | 70 | 5.0 | SDS | 4 times | 6.0 | 400 | 1.02 | 1.72 |
| Experiment 6 | P(3HB-co-4HB) | 25.1 | 56.3 | 10.95 | 70 | 7.0 | SDS | 4 times | 6.0 | 200 | 1.02 | 1.72 |
| Experiment 7 | P(3HB-co-4HB) | 24.7 | 63.0 | 9.13 | 70 | 6.0 | SDS | 4 times | 6.0 | 50 | 1.01 | 1.68 |
| Experiment 8 | P(3HB-co-4HB) | 24.7 | 63.0 | 9.13 | 70 | 6.0 | SDS | 4 times | 6.0 | 30 | 1.01 | 1.68 |
| Experiment 9 | P(3HB-co-4HB) | 24.7 | 63.0 | 9.13 | 70 | 6.0 | SDS | 1 time | 6.0 | 200 | 1.01 | 1.68 |
| Experiment 10 | P(3HB-co-4HB) | 25.1 | 56.3 | 10.95 | 70 | 7.5 | SDS | 4 times | 6.0 | 200 | 1.02 | 1.72 |

**[Table 2]**

| | | | | | First run of stirring step | | | | | | | |
|---|---|---|---|---|---|---|---|---|---|---|---|---|
| | Initial required stirring power (W/m3) | Final viscosity (cP) | Final required stirring power (W/m3) | before purification (10K) | Collection rate (%) | 90% size (µm) | Median size (µm) | 10% size (µm) | Variation coefficient (%) | Bulk density (g/mL) | Mw after purification (10K) | Total nitrogen concentration (ppm) |
| Experiment 1 | 120 | 1.21 | 111 | 153 | 100 | 3251 | 2586 | 1994 | 19.3 | 0.091 | 70.2 | 33 |
| Experiment 2 | 18 | 1.21 | 16 | 153 | 100 | 2857 | 1860 | 1054 | 36.3 | 0.088 | 79.0 | 81 |
| Experiment 3 | 831 | 1.08 | 765 | 187 | 101 | 2593 | 1799 | 1184 | 31.2 | 0.089 | 65.3 | 47 |
| Experiment 4 | 831 | 1.08 | 765 | 187 | 103 | 2767 | 1857 | 1214 | 31.9 | 0.100 | 59.2 | 380 |
| Experiment 5 | 831 | 1.08 | 765 | 187 | 100 | 2527 | 1752 | 1218 | 29.9 | 0.088 | 61.9 | 62 |
| Experiment 6 | 121 | 1.08 | 109 | 187 | 100 | 2575 | 1511 | 810 | 43.1 | 0.142 | 65.5 | 51 |
| Experiment 7 | 2.7 | 1.21 | 2.5 | 153 | 101 | 2150 | 1077 | 425 | 58.5 | 0.135 | 81.0 | 53 |
| Experiment 8 | 0.7 | 1.21 | 0.6 | 153 | 100 | 1234 | 786 | 358 | 43.3 | 0.175 | 79.0 | 39 |
| Experiment 9 | 120 | 1.21 | 111 | 153 | 0.6 | - | - | - | - | - | - | - |
| Experiment 10 | 121 | 1.08 | 109 | 187 | 62 | 2552 | 1508 | 751 | 44.3 | 0.157 | 64.7 | 172 |

**[Table 3]**

| Second run of stirring step | | | | | | |
|---|---|---|---|---|---|---|
| Collection rate (%) | 90% size (µm) | Median size (µm) | 10% size (µm) | Variation coefficient (%) | Mw after purification (10K) | Total nitrogen concentration (ppm) |
| 100 | 3187 | 2487 | 1800 | 22.1 | 66.1 | 5.8 |

## Claims

1. A method for producing an aggregate of polyhydroxyalkanoic acid, the method comprising:
a culture step of culturing a microorganism that intracellularly accumulates polyhydroxyalkanoic acid; and
a stirring step of adding hydrogen peroxide and a surfactant to the obtained culture solution, followed by stirring under a condition of pH controlled at 8 or less at a required stirring power, as calculated from the Kamei-Hiraoka equation, of 2 W/m³ or more, wherein the surfactant is added in an amount of twice or more by weight relative to the microbial cell weight, which is calculated by subtracting the dry weight of polyhydroxyalkanoic acid from the dry cell weight of the microorganism including polyhydroxyalkanoic acid.

2. The method according to claim 1, further comprising a collection step of collecting the aggregate of polyhydroxyalkanoic acid after the stirring step.

3. The method according to claim 1 or 2, further comprising a sterilization step of performing sterilization treatment of the microorganism by heat treatment after the culture step.

4. The method according to claim 1 or 2, wherein the pH in the stirring step is controlled at from 4.0 to 7.0.

5. The method according to claim 1 or 2, wherein the surfactant is sodium dodecyl sulfate.

6. The method according to claim 1 or 2, wherein the amount of hydrogen peroxide added is 2 to 10% by weight.

7. The method according to claim 1 or 2, wherein the required stirring power calculated from the Kamei-Hiraoka equation is 5 W/m³ or more for 5 hours or longer after addition of hydrogen peroxide and the surfactant.

8. The method according to claim 1 or 2, wherein the required stirring power calculated from the Kamei-Hiraoka equation is 10 W/m³ or more for 5 hours or longer after addition of hydrogen peroxide and the surfactant.

9. The method according to claim 1 or 2, wherein a liquid temperature in the stirring step is 40°C to 100°C.

10. The method according to claim 1 or 2, wherein a liquid temperature in the stirring step is 50°C to 80°C.

11. The method according to claim 1 or 2, wherein a median size of the obtained aggregate of polyhydroxyalkanoic acid is 1000 µm or more.

12. The method according to claim 1 or 2, wherein a total nitrogen concentration of the obtained aggregate of polyhydroxyalkanoic acid is 500 ppm or lower.

13. The method according to claim 1 or 2, wherein the polyhydroxyalkanoic acid comprises 3-hydroxybutyric acid as a monomer unit.

14. The method according to claim 1 or 2, wherein the polyhydroxyalkanoic acid is a copolymer comprising 3-hydroxybutyric acid and 4-hydroxybutyric acid as monomer units.

15. The method according to claim 1 or 2, wherein the microorganism is a microorganism of a genus selected from the group consisting of the genus *Cupriavidus,* the genus *Alcaligenes,* the genus *Ralstonia,* the genus *Delftia,* the genus *Comamonas,* the genus *Hydrogenophaga,* the genus *Burkholderia,* the genus *Escherichia,* the genus *Azotobacter,* the genus *Methylobacterium,* the genus *Paracoccus,* the genus *Pseudomonas,* the genus *Acinetobacter,* the genus *Aeromonas,* the genus *Allochromatium,* the genus *Azorhizobium,* the genus *Bacillus,* the genus *Caulobacter,* the genus *Chromobacterium,* the genus *Ectothiorhodospira,* the genus *Klebsiella,* the genus *Nocardia,* the genus *Rhodobacter,* the genus *Rhodococcus,* the genus *Rhodospirillum,* the genus *Rickettsia,* the genus *Sinorhizobium,* the genus *Sphingomonas,* the genus *Synechocystis,* the genus *Thiococcus,* the genus *Thiocystis,* the genus *Vibrio,* and the genus *Wautersia.*

16. The method according to claim 15, wherein the microorganism is *Cupriavidus necator.*

17. A polyhydroxyalkanoic acid aggregate having a median size of 1000 µm or more and a bulk density of 0.06 g/mL to 0.20 g/mL in a dry state.

18. The polyhydroxyalkanoic acid aggregate according to claim 17, wherein the bulk density in a dry state is 0.08 g/mL to 0.10 g/mL.

19. The polyhydroxyalkanoic acid aggregate according to claim 17 or 18, wherein a total nitrogen concentration is 500 ppm or lower.
